# EUROPEAN PATENT SPECIFICATION

(11) **EP 3 618 888 B1**
(45) Date of publication and mention of the grant of the patent: **24.06.2026**
(21) Application number: 17908672.3
(22) Date of filing: 05.05.2017
(51) Int. Cl.: A61M 1/28

(54) **PERITONEAL DIALYSIS CONCENTRATE, PERITONEAL DIALYSIS BAG AND SET FOR CONTINUOUS AMBULATORY PERITONEAL DIALYSIS OR AUTOMATED PERITONEAL DIALYSIS**
PERITONEALDIALYSEKONZENTRAT, PERITONEALDIALYSEBEUTEL UND SET ZUR KONTINUIERLICHEN AMBULANTEN PERITONEALDIALYSE ODER AUTOMATISIERTEN PERITONEALDIALYSE
CONCENTRÉ DE DIALYSE PÉRITONÉALE, POCHE DE DIALYSE PÉRITONÉALE ET ENSEMBLE DE DIALYSE PÉRITONÉALE AMBULATOIRE CONTINUE OU DIALYSE PÉRITONÉALE AUTOMATISÉE

(43) Date of publication of application: 11.03.2020
(73) Proprietor: Fresenius Medical Care Deutschland GmbH, 61352 Bad Homburg (DE); Fresenius Medical Care Holdings, Inc., Waltham, MA 02451-1457 (US)
(72) Inventor: LU, Aaron, Shanghai 201908 (CN); LIU, Tao, Shanghai 201101 (CN); MA, Zhengxin, Shanghai 201612 (CN); GONG, Mingtao, Shanghai 200135 (CN); LASHER, Richard Allen, Knoxville, TN 37932 (US); CAO, Haijun, Wuxi Jiangsu 214023 (CN)
(74) Representative: Lorenz Seidler Gossel Part. mbB
(86) International application number: PCT/CN2017/083196
(87) International publication number: WO 2018/201443

(56) References cited:
- EP-A1- 3 025 738
- EP-B1- 1 033 984
- EP-B1- 2 231 099
- EP-B1- 2 512 546
- WO-A1-2013/055283
- CN-A- 102 770 167
- CN-A- 103 458 937
- CN-A- 104 582 750
- CN-A- 106 581 053
- US-A1- 2005 020 507
- US-A1- 2006 226 080
- US-A1- 2012 288 572

## Description

### Technical Field

The present invention relates to a peritoneal dialysis concentrate and to sets for an automated peritoneal dialysis comprising respectively a single-chamber and a dual chamber bag, wherein said bags comprise the previously mentioned peritoneal dialysis concentrate.

### Background Art

A peritoneal dialysis (PD) is a procedure for removing toxic substances and metabolites normally removed by the kidneys, and for aiding in regulation of fluid and electrolyte balance. The procedure is accomplished by infusing a PD solution through a conduit into the peritoneal cavity. Osmosis and diffusion occur across the peritoneal membrane between plasma of the patient and the solution. In order to realize the whole process, PD products are developed with sterilized PD solutions and packaging.

WO 2013/055283 refers to such products. It discloses an all-in-one concentrate for a PD solution.

A continuous ambulatory peritoneal dialysis is one form of PD that allows the patient to exchange the PD solutions 3-4 times per day. In addition, there also is an automated peritoneal dialysis that can automatically accomplish the peritoneal dialysis procedure.

For executing the CAPD and APD, some sets for CAPD and APD are developed which are used for infusing the PD solution from a solution bag into the peritoneal cavity.

The PD solution is usually contained in an un-concentrated state in the solution bag. That is to say, the PD solution can be infused into the peritoneal cavity without any additional dilution process.

However, due to the un-concentrated state of the solution, the solution bag filled with the PD solution has a relatively large volume, which requires a larger storage space and leads to a high cost. Moreover, it is not easy to transport such a solution bag.

### Summary of the Invention

In view of the problems existing in the prior art, an object of the present invention is to provide an all-in-one peritoneal dialysis concentrate.

The invention is a peritoneal dialysis concentrate comprising an all-in-one concentrate, the all-in-one concentrate containing at least one electrolyte salt, at least one buffering agent, at least one osmosis agent and a pH adjusting agent, wherein the osmosis agent comprises at least one of dextrose, icodextrin, glucose polymers, sorbitol or fructose, further wherein the all-in-one concentrate is configured to be diluted to obtain a peritoneal dialysis solution with a pH in a range of 4.0-8.0 and suitable for PD treatment, and wherein the diluting solution is water for injection or water for injection directly generated from pure water or reverse osmosis water, characterized in that the pH of the all-in-one concentrate is in a range of 4.0-8.0

The present invention also relates to a set for a continuous ambulatory peritoneal dialysis or an automated peritoneal dialysis, wherein the set comprises a single-chamber peritoneal dialysis bag, which contains the peritoneal dialysis concentrate defined above, wherein the single-chamber peritoneal dialysis bag comprises: a single chamber for containing a peritoneal dialysis concentrate; a diluting solution tubing fluidly connected with the chamber for receiving a diluting solution from a diluting solution source; and a connecting tubing fluidly connected with the chamber, wherein the diluting solution can be filled into the chamber via the diluting solution tubing to dilute or dissolve the peritoneal dialysis concentrate and obtain a peritoneal dialysis solution suitable for a peritoneal dialysis treatment via the connecting tubing.

The present invention also relates to a set for a continuous ambulatory peritoneal dialysis or an automated peritoneal dialysis, wherein the set comprises a dual-chamber peritoneal dialysis bag comprising: a first chamber containing the peritoneal dialysis concentrate defined above; a second chamber separated from the first chamber by a peelable welding line; a diluting solution tubing fluidly connected with the second chamber which is empty for receiving a diluting solution from a diluting solution source; and a connecting tubing fluidly connected with the second chamber, wherein the diluting solution can be filled into the second chamber via the diluting solution tubing to break the peelable welding line such that the peritoneal dialysis concentrate is diluted or dissolved to obtain a peritoneal dialysis solution suitable for a peritoneal dialysis treatment via the connecting tubing.

According to an optional embodiment, the pH adjusting agent is hydrochloric acid. According to an optional embodiment, the electrolyte salt includes one selected from a group consisting of salt of sodium, salt of calcium, salt of magnesium and any mixtures thereof; and/or the buffering agent is an alkaline buffering agent including one selected from a group consisting of sodium lactate, sodium bicarbonate and any mixtures thereof; and/or the osmosis agent includes one selected from a group consisting of amino acids, small molecule crystal glycerin, macromolecule gel, cationic polymer, polypeptide and any mixtures thereof.

According to an optional embodiment, the peritoneal dialysis concentrate can be diluted 2-20 times to obtain the peritoneal dialysis solution. According to an optional embodiment, the peritoneal dialysis concentrate can be diluted 8-12 times.

According to an optional embodiment, the peritoneal dialysis solution can be used for a continuous ambulatory peritoneal dialysis or an automated peritoneal dialysis treatment.

In a further aspect, provided is a set for a continuous ambulatory peritoneal dialysis, wherein the set comprises the single-chamber peritoneal dialysis bag or the dual-chamber peritoneal dialysis bag, which contains the peritoneal dialysis concentrate.

### Brief Description of the Drawings

The present invention and advantages thereof will be further understood by reading the following detailed description of some preferred exemplary embodiments with reference to the drawings in which:
Fig. 1 shows an exemplary single-chamber bag filled with a PD concentrate.
Fig. 2 shows an exemplary dual-chamber bag filled with the PD concentrate in one of two chambers.
Fig. 3 shows an exemplary multi-chamber bag filled with a first concentrate part and a second concentrate part in a separate manner.

### Detailed Description of Preferred Embodiments

Some exemplary embodiments of the present invention and examples which are related although not part of the invention will be described hereinafter in more details with reference to the drawings to better understand the basic concept of the present invention.

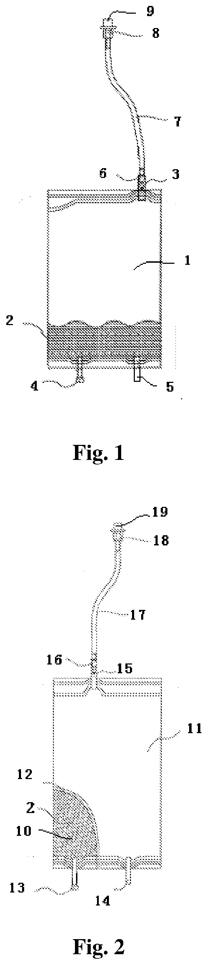

Firstly, an exemplary single-chamber bag for holding a concentrated PD solution will be described with reference to Fig. 1.

As shown in Fig. 1, the single-chamber bag mainly comprises a chamber 1 to be filled with a mixed concentrate 2, a diluting solution tubing 3 to be connected to a diluting solution filling machine (not shown), an injection port 4 allowing for infusion of a desired substance, such as a pharmaceutical agent, into the chamber 1, and a connecting tubing 5 to be connected to a corresponding component of a set for CAPD/APD (not shown).

As also can be seen from Fig. 1, the diluting solution tubing 3 is fluidly communicated with the chamber 1 and connected to the diluting solution filling machine via a frangible plug 6, a diluting solution transfer tubing 7 and a diluting solution connector 8. Specifically, the diluting solution tubing 3 is connected with the frangible plug 6 which can prevent the concentrate 2 from flowing out of the chamber 1 in an initial state, and the diluting solution transfer tubing 7 is connected between the frangible plug 6 and the diluting solution connector 8 to be connected with the diluting solution filling machine. The diluting solution connector 7 can be covered by a cover 9 to avoid contamination.

According to an example, the concentrate 2 in the single-chamber bag may contain three constituents including: an electrolyte salt, a buffering agent and an osmosis agent.

According to an example, the electrolyte salt may be at least one salt of sodium, salt of calcium and salt of magnesium.

According to an example, the buffering agent may be at least one of sodium lactate, sodium bicarbonate and other alkaline buffering solution.

According to an example, the osmosis agent may be at least one of dextrose, icodextrin, glucose polymers, amino acids, small molecule crystal glycerin, sorbitol, fructose, macromolecule gel, cationic polymer and polypeptide.

It should be understood by a skilled person in the art that the concentrate 2 also may contain other additional substances as desired.

According to the present invention, the pH of the concentrate 2 must be adjusted to be in a range of 4.0-8.0. The pH of the concentrate 2 is adjusted by adding a pH adjusting agent, such as hydrochloric acid. The pH of 4.0-8.0 will ensure that a lower level of glucose degradation products (GDPs) is generated and a final PD solution obtained by diluting the concentrate 2 using a diluting solution has a desired pH value.

The diluting solution is one of pure water, reverse osmosis water, water for injection (WFI) and sterile water for injection. In use, the cover 9 is detached from the diluting solution connector 8 and then the diluting solution connector 8 is connected to the diluting solution filling machine. The frangible plug 6 is broken and the diluting solution is filled into the chamber 1 from the diluting solution filling machine via the diluting solution transfer tubing 7, the broken frangible plug 6 and the diluting solution tubing 3 and mixed with the concentrate 2. After filling of the diluting solution, the diluting solution transfer tubing 7 is sealed and the diluting solution connector 8 is disconnected from the diluting solution filling machine.

The concentrate 2 may be diluted 2-20 times, such as 16 times using the diluting solution and the final PD solution may be used for CAPD or APD. For example, the concentrate 2 may be diluted to be the final PD solution of 1-3 L which is suitable for CAPD or may be diluted to be the final PD solution of 4-15 L which is suitable for APD.

Table 1 shows an exemplary formulation design of the concentrate 2 for the single-chamber bag.

**Table 1**

| Constituent | Concentration (g/L) |
|---|---|
| NaCl | 11.10-140.26 |
| CaCl₂-2H₂O | 0-7.35 |
| MgCl₂-6H₂O | 0.10-6.10 |
| Sodium lactate | 6.72-134.47 |
| Glucose polymers | 9.01-900.81 |
| Hydrochloric acid | suitable quantity |

The concentrate 2 shown in Table 1 may be diluted so as to obtain a final PD solution as shown in Table 2.

**Table 2**

| Constituent | Concentration (mmol/L) |
|---|---|
| Na⁺ | 125-150 |
| Ca²⁺ | 0-2.5 |
| Mg²⁺ | 0.25-1.5 |
| Cl⁻ | 90-120 |
| Lactate | 30-60 |
| Glucose polymers | 25-250 |

Table 3 shows 6-month (6M) accelerated stability testing results of three concentrates having respective pH of 4.7, 5.0 and 5.3. In Table 3, the three concentrates are denoted as F1, F2 and F3 and 0M, 1M, 2M, 3M and 6M denote an initial detection and detections after acceleration of 1 month, 2 months, 3 months and 6 months respectively. For example, F1-1M denotes that a detection is carried out on the F1 concentrate after acceleration of 1 month.

**Table 3**

| Tests | 3-DG (uM) | Glx (uM) | mGlx (uM) | FoA (uM) | 5-HMF (uM) | AcA (uM) | FurA (uM) | Total GDP (uM) | pH | pH (Diluted) |
|---|---|---|---|---|---|---|---|---|---|---|
| F1-0M | 64 | 6 | 2 | 6 | 10 | 4 | 0 | 93 | 4.6 | N/A |
| F1-1M | 62 | 7 | 2 | 3 | 29 | 10 | 0 | 113 | 4.6 | N/A |
| F1-2M | 43 | 7 | 2 | 4 | 35 | 15 | 2 | 105 | 4.6 | N/A |
| F1-3M | 42 | 7 | 2 | 2 | 18 | 8 | 1 | 79 | 4.6 | N/A |
| F1-6M | 33.3 | 6.1 | 0.4 | 2.6 | 43.7 | 14.1 | 3.3 | 100.3 | 4.6 | 5.0 |
| F2-0M | 64 | 6 | 2 | 3 | 19 | 9 | 0 | 104 | 4.7 | N/A |
| F2-1M | 61 | 6 | 3 | 3 | 20 | 11 | 0 | 105 | 4.7 | N/A |
| F2-2M | 46 | 7 | 3 | 4 | 27 | 15 | 1 | 101 | 4.7 | N/A |
| F2-3M | 43 | 6 | 2 | 2 | 14 | 8 | 0 | 76 | 4.8 | N/A |
| F2-6M | 39.7 | 5.1 | 0.7 | 2.3 | 35.5 | 10.5 | 2.1 | 93.8 | 4.7 | 5.1 |
| F3-0M | 65 | 5 | 4 | 4 | 7 | 10 | 0 | 96 | 4.9 | N/A |
| F3-1M | 68 | 5 | 5 | 4 | 10 | 12 | 0 | 104 | 4.9 | N/A |
| F3-2M | 49 | 5 | 4 | 4 | 14 | 17 | 2 | 94 | 4.8 | N/A |
| F3-3M | 48 | 6 | 4 | 2 | 9 | 12 | 1 | 80 | 4.9 | N/A |
| F3-6M | 46.8 | 4.6 | 1.2 | 2.2 | 21.3 | 14.3 | 4.3 | 90.4 | 4.8 | 5.2 |

The testing results show that the concentrate 2 has good properties, in particular a lower level of GDP. Obviously, the final PD solution will have good properties.

The concentrate 2 may also be filled into a dual-chamber bag shown in Fig. 2. As shown in Fig. 2, the dual-chamber bag comprises a first chamber 10 and a second chamber 11. The first chamber 10 and the second chamber 11 are separated from each other, preferably by a peelable welding line 12. The concentrate 2 is filled into the first chamber 10 and an injection port 13 allows for infusion of a desired substance, such as a pharmaceutical agent, into the first chamber 10. A connecting tubing 14 is to be connected to the corresponding component of the set for CAPD/APD. The second chamber 11 is empty before use.

As also can be seen from Fig. 2, a diluting solution tubing 15 is fluidly communicated with the second chamber 11 and connected with the diluting solution filling machine via a frangible plug 16, a diluting solution transfer tubing 17 and a diluting solution connector 18. Specifically, the diluting solution tubing 15 is connected with the frangible plug 16 which seals the second chamber 11 in an initial state, and the diluting solution transfer tubing 17 is connected between the frangible plug 16 and the diluting solution connector 18 to be connected with the diluting solution filling machine. The diluting solution connector 18 can be covered by a cover 19 to avoid contamination.

In use, the diluting solution is filled into the second chamber 11 from the diluting solution filling machine and then the diluting solution transfer tubing 17 is sealed and the diluting solution connector 18 is disconnected from the diluting solution filling machine. The peelable welding line 12 may be opened by a pressure of the diluting solution during filling of the diluting solution and/or by pressing the dual-chamber bag after sealing of the diluting solution transfer tubing 17. Upon opening of the peelable welding line 12, the concentrate 2 is mixed with the diluting solution such that the concentrate 2 may be diluted 2-20 times, such as 16 times so as to obtain the final PD solution.

The concentrate 2 is stored in a relatively small space and thus the concentrate 2 will contact with a gas (such as air) within the first chamber 10 to the least extent, which is beneficial.

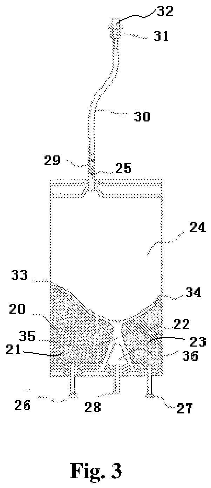

Now, an exemplary multi-chamber bag not part of the invention will be described with reference to Fig. 3.

As shown in Fig. 3, the multi-chamber bag mainly comprises a first chamber 20 to be filled with a first concentrate part 21, a second chamber 22 to be filled with a second concentrate part 23, a third chamber 24 being empty before use, a diluting solution tubing 25 to be connected to the diluting solution filling machine, a first injection port 26 allowing for infusion of a first desired substance, such as a pharmaceutical agent, into the first chamber 20, a second injection port 27 allowing for infusion of a second desired substance, such as a pharmaceutical agent, into the second chamber 22, and a connecting tubing 28 to be connected to the corresponding component of the set for CAPD/APD.

As also can be seen from Fig. 3, the diluting solution tubing 25 is fluidly communicated with the third chamber 24 and connected with the diluting solution filling machine via a frangible plug 29, a diluting solution transfer tubing 30 and a diluting solution connector 31 in the same manner as in the single-chamber bag shown in Fig, 1. The diluting solution connector 31 can be covered by a cover 32 to avoid contamination.

The first chamber 20, the second chamber 22 and the third chamber 24 are separated from each other, preferably by respective peelable welding lines 33, 34 and 35.

In the embodiment shown in Fig. 3, the multi-chamber bag preferably further comprises a fourth chamber 36 which is empty before use. The connecting tubing 28 is fluidly communicated with the fourth chamber 36. The fourth chamber 36 is separated from the first, second and third chambers, preferably by respective peelable welding lines.

As an alternative embodiment, the fourth chamber 36 may also be omitted and the connecting tubing 28 is fluidly communicated with the third chamber 24.

The first concentrate part 21 may contain an electrolyte salt and an osmosis agent, and the second concentrate part 23 may contain a buffering agent, but not an electrolyte salt and an osmosis agent. Preferably, the first concentrate part 21 may further contain a buffering agent, such as one of sodium lactate, sodium bicarbonate and other alkaline buffering solution.

The first concentrate part 21 and/or the second concentrate part 23 may be in a form of liquid or powder.

The electrolyte salt, the buffering agent and the osmosis agent used in the single-chamber bag are also suitable for the multi-chamber bag.

The pH of the first concentrate part 21 may be adjusted to be in an optimal pH range of 1.0-4.0, preferably by adding a pH adjusting agent, such as hydrochloric acid. The pH of 1.0-4.0 will ensure that a lower level of GDP is generated.

The second concentrate part 23 may be designed as a buffering solution containing a buffering agent and having the pH of 6.5-8.0, to ensure that the final PD solution has a suitable pH range of 6.5-8.0. For example, the second concentrate part 23 may contain at least one of sodium lactate, sodium bicarbonate and other alkaline buffering solution.

In use, a diluting solution is filled into the third chamber 24 from the diluting solution filling machine, and then the diluting solution transfer tubing 30 is sealed and the diluting solution connector 31 is disconnected from the diluting solution filling machine. Firstly, the peelable welding lines 33 and 34 may be opened by a pressure of the diluting solution during filling of the diluting solution and/or by pressing the multi-chamber bag after sealing of diluting solution transfer tubing 30. Upon opening of the peelable welding lines 33 and 34, the first concentrate part 21 and the second concentrate part 23 each are mixed with the diluting solution and then mixed with each other to obtain the final PD solution. The peelable welding line 35 may be opened by further pressing the multi-chamber bag such that the final PD solution flows into the fourth chamber 36 and then to a patient via the connecting tubing 28.

For the multi-chamber bag, the diluting solution may be at least one of pure water, reverse osmosis water, WFI, sterile water for injection, etc.

As shown in Fig. 3, the peelable welding line 35 is wider than the peelable welding lines 33 and 34 such that the peelable welding lines 33 and 34 are opened before the peelable welding line 35. For a skilled person in the art, it is also possible to control the order of opening of the peelable welding lines and thus the order of mixing by designing shape and welding strength of the peelable welding lines.

Table 4 shows an exemplary formulation design of the first concentrate part 21 and the second concentrate part 23 for the multi-chamber bag.

**Table 4**

| Constituent | First concentrate part (g/L) | Second concentrate part (g/L) |
|---|---|---|
| NaCl | 11.10-140.26 | N/A |
| CaCl₂-2H₂O | 0-7.35 | N/A |
| MgCl₂-6H₂O | 0.10-6.10 | N/A |
| Glucose polymers | 9.01-900.81 | N/A |
| Sodium lactate | 0-134.47 | 0-134.47 |
| Sodium bicarbonate | N/A | 0-100.81 |
| Hydrochloric acid | suitable quantity | N/A |

The first concentrate part 21 and the second concentrate part 23 may be mixed and diluted to obtain the final PD solution as shown in Table 5.

**Table 5**

| Constituent | Concentration (mmol/L) |
|---|---|
| Na⁺ | 125-150 |
| Ca²⁺ | 0-2.5 |
| Mg²⁺ | 0.25-1.5 |
| Cl⁻ | 90-120 |
| Dextrose | 25-250 |
| Lactate | 0-60 |
| HCO₃⁻ | 0-60 |

Table 6 shows 6-month accelerated stability testing results of three respective mixtures of the first concentrate parts having respective pH of 3.0, 2.5 and 2.5 and the second concentrate parts having respective pH of 8.0, 8.0 and 8.1. The same denotations used in Table 6 have the same meaning as in Table 3.

**Table 6**

| Tests | 3-DG (uM) | Glx (uM) | mGlx (uM) | FoA (uM) | 5-HMF (uM) | AcA (uM) | FurA (uM) | Total GDP (uM) |
|---|---|---|---|---|---|---|---|---|
| F1-0M | 27.7 | 0.8 | 0.0 | 0.0 | 31.6 | 0.0 | 0.0 | 60 |
| F1-1M | 22.3 | 0.7 | 0.0 | 1.2 | 37.2 | 0.3 | 0.0 | 62 |
| F1-2M | 19.0 | 1.3 | 0.0 | 1.1 | 40.5 | 3.3 | 0.0 | 65 |
| F1-3M | 18.8 | 1.1 | 0.0 | 2.4 | 41.4 | 0.0 | 0.0 | 64 |
| F1-6M | 1.8 | 5.4 | 0.0 | 1.4 | 38.5 | 0.0 | 2.5 | 50 |
| F2-0M | 18.2 | 0.5 | 0.0 | 0.0 | 23.2 | 0.0 | 0.0 | 42 |
| F2-1M | 13.4 | 0.4 | 0.0 | 1.4 | 27.8 | 1.3 | 0.0 | 44 |
| F2-2M | 13.4 | 1.2 | 0.0 | 1.3 | 30.3 | 3.3 | 0.0 | 50 |
| F2-3M | 10.6 | 0.9 | 0.0 | 2.5 | 29.3 | 0.0 | 0.0 | 43 |
| F2-6M | 4.5 | 4.8 | 0.0 | 1.7 | 30.9 | 4.1 | 3.2 | 49 |
| F3-0M | 18.2 | 0.5 | 0.0 | 0.0 | 23.2 | 0.0 | 0.0 | 42 |
| F3-1M | 15.1 | 0.5 | 0.0 | 1.4 | 25.9 | 0.2 | 0.0 | 43 |
| F3-2M | 11.3 | 1.4 | 0.0 | 1.7 | 33.4 | 6.1 | 0.0 | 54 |
| F3-3M | 12.0 | 1.2 | 0.0 | 2.6 | 28.7 | 0.0 | 0.0 | 44 |
| F3-6M | 4.6 | 5.5 | 0.0 | 1.9 | 30.9 | 3.2 | 3.1 | 49 |

The testing results show that the mixture of the first concentrate part 21 and the second concentrate part 23 has good properties, in particular a lower level of GDP. Obviously, the final PD solution will have good properties.

The first concentrate part 21 and the second concentrate part 23 may be diluted 2-20 times, such as 12.5 times, using the diluting solution and the final PD solution may be used for CAPD or APD.

It should be understood by a skilled person in the art that dilution times or concentration times of the concentrate may be determined according to different treatments.

In addition, the multi-chamber bag may also comprise more chambers such that the PD concentrate can be divided into more parts being contained in respective chambers of the multi-chamber bag.

The basic concept of the present invention is to provide an all-in-one PD concentrate and a set for CAPD/APD using the PD concentrate. The PD concentrate and the corresponding set for CAPD/APD can allow for saving logistic cost, easily transporting and only requiring minimum storage space.

## Claims

1. A peritoneal dialysis concentrate comprising an all-in-one concentrate, the all-in-one concentrate containing at least one electrolyte salt, at least one buffering agent, at least one osmosis agent and a pH adjusting agent, wherein the osmosis agent comprises at least one of dextrose, icodextrin, glucose polymers, sorbitol or fructose, further wherein the all-in-one concentrate is configured to be diluted to obtain the peritoneal dialysis solution with a pH in a range of 4.0-8.0 and suitable for peritoneal dialysis treatment,
and wherein the diluting solution is water for injection or water for injection directly generated from pure water or reverse osmosis water,
**characterized in that** the pH of the all-in-one concentrate is in a range of 4.0-8.0.

2. The peritoneal dialysis concentrate according to claim 1, wherein the pH adjusting agent is hydrochloric acid.

3. The peritoneal dialysis concentrate according to claim 1 or 2, wherein:
the electrolyte salt includes one selected from a group consisting of salt of sodium, salt of calcium, salt of magnesium and any mixtures thereof; and/or the buffering agent is an alkaline buffering agent including one selected from a group consisting of sodium lactate, sodium bicarbonate and any mixtures thereof; and/or
the osmosis agent includes one selected from a group consisting of amino acids, small molecule crystal glycerin, macromolecule gel, cationic polymer, polypeptide and any mixtures thereof.

4. The peritoneal dialysis concentrate according to any one of claims 1 to 3, wherein:
the peritoneal dialysis concentrate can be diluted 2-20 times to obtain the peritoneal dialysis solution.

5. The peritoneal dialysis concentrate according to claim 4, wherein the peritoneal dialysis concentrate can be diluted 8-12 times.

6. The peritoneal dialysis concentrate according to any one of claims 1 to 5, wherein
the peritoneal dialysis solution can be used for a continuous ambulatory peritoneal dialysis or an automated peritoneal dialysis treatment.

7. A set for a continuous ambulatory peritoneal dialysis or an automated peritoneal dialysis, wherein the set comprises a single-chamber peritoneal dialysis bag, which contains the peritoneal dialysis concentrate according to any one of claims 1 to 6, wherein the single-chamber peritoneal dialysis bag comprises:
a single chamber for containing a peritoneal dialysis concentrate;
a diluting solution tubing fluidly connected with the chamber for receiving a diluting solution from a diluting solution source; and
a connecting tubing fluidly connected with the chamber,
wherein the diluting solution can be filled into the chamber via the diluting solution tubing to dilute or dissolve the peritoneal dialysis concentrate and obtain a peritoneal dialysis solution suitable for a peritoneal dialysis treatment via the connecting tubing.

8. A set for a continuous ambulatory peritoneal dialysis or an automated peritoneal dialysis, wherein the set comprises a dual-chamber peritoneal dialysis bag comprising:
a first chamber containing the peritoneal dialysis concentrate according to any one of claims 1 to 6;
a second chamber separated from the first chamber by a peelable welding line;
a diluting solution tubing fluidly connected with the second chamber which is empty for receiving a diluting solution from a diluting solution source; and
a connecting tubing fluidly connected with the second chamber,
wherein the diluting solution can be filled into the second chamber via the diluting solution tubing to break the peelable welding line such that the peritoneal dialysis concentrate is diluted or dissolved to obtain a peritoneal dialysis solution suitable for a peritoneal dialysis treatment via the connecting tubing.

## Patentansprüche

1. Peritonealdialysekonzentrat, umfassend ein All-in-one-Konzentrat, wobei das All-in-one-Konzentrat mindestens ein Elektrolytsalz, mindestens einen Pufferstoff, mindestens ein Osmosemittel und ein pH-Einstellmittel enthält, wobei das Osmosemittel mindestens eines von Dextrose, Icodextrin, Glucosepolymeren, Sorbitol oder Fructose umfasst, wobei ferner das All-in-one-Konzentrat so ausgelegt ist, dass es verdünnt wird, um die Peritonealdialyselösung mit einem pH-Wert in einem Bereich von 4,0-8,0 zu erhalten, und für eine Peritonealdialysebehandlung geeignet ist,
und wobei die Verdünnungslösung Wasser für Injektionszwecke oder Wasser für Injektionszwecke ist, das direkt aus Reinstwasser oder Umkehrosmosewasser erzeugt wird, **dadurch gekennzeichnet, dass**
der pH-Wert des All-in-one-Konzentrats in einem Bereich von 4,0-8,0 liegt.

2. Peritonealdialysekonzentrat nach Anspruch 1, wobei das pH-Einstellmittel Salzsäure ist.

3. Peritonealdialysekonzentrat nach Anspruch 1 oder 2, wobei:
das Elektrolytsalz eines umfasst, das aus einer Gruppe ausgewählt ist, bestehend aus Natriumsalz, Calciumsalz, Magnesiumsalz und beliebigen Mischungen davon;
und/oder
der Pufferstoff ein alkalischer Pufferstoff ist, der eines umfasst, das aus einer Gruppe ausgewählt ist, bestehend aus Natriumlactat, Natriumhydrogencarbonat und beliebigen Mischungen davon;
und/oder
das Osmosemittel eines umfasst, das aus einer Gruppe ausgewählt ist, bestehend aus Aminosäuren, niedermolekularem kristallinem Glycerin, makromolekularem Gel, kationischem Polymer, Polypeptid und beliebigen Mischungen davon.

4. Peritonealdialysekonzentrat nach einem der Ansprüche 1 bis 3, wobei:
das Peritonealdialysekonzentrat 2- bis 20-fach verdünnt werden kann, um die Peritonealdialyselösung zu erhalten.

5. Peritonealdialysekonzentrat nach Anspruch 4, wobei das Peritonealdialysekonzentrat 8- bis 12-fach verdünnt werden kann.

6. Peritonealdialysekonzentrat nach einem der Ansprüche 1 bis 5, wobei die Peritonealdialyselösung für eine kontinuierliche ambulante Peritonealdialyse oder eine automatisierte Peritonealdialysebehandlung verwendet werden kann.

7. Set für eine kontinuierliche ambulante Peritonealdialyse oder eine automatisierte Peritonealdialyse, wobei das Set einen Einkammer-Peritonealdialysebeutel umfasst, der das Peritonealdialysekonzentrat nach einem der Ansprüche 1 bis 6 enthält, wobei der Einkammer-Peritonealdialysebeutel umfasst:
eine einzelne Kammer zum Enthalten eines Peritonealdialysekonzentrats;
einen Verdünnungslösungsschlauch, der strömungsverbunden mit der Kammer ist, zum Aufnehmen einer Verdünnungslösung aus einer Verdünnungslösungsquelle; und einen Verbindungsschlauch, der strömungsverbunden mit der Kammer ist,
wobei die Verdünnungslösung über den Verdünnungslösungsschlauch in die Kammer eingefüllt werden kann, um das Peritonealdialysekonzentrat zu verdünnen oder zu lösen und über den Verbindungsschlauch eine für eine Peritonealdialysebehandlung geeignete Peritonealdialyselösung zu erhalten.

8. Set für eine kontinuierliche ambulante Peritonealdialyse oder eine automatisierte Peritonealdialyse, wobei das Set einen Zweikammer-Peritonealdialysebeutel umfasst, der umfasst:
eine erste Kammer, die das Peritonealdialysekonzentrat nach einem der Ansprüche 1 bis 6 enthält;
eine zweite Kammer, die von der ersten Kammer durch eine abziehbare Schweißnaht getrennt ist;
einen Verdünnungslösungsschlauch, der strömungsverbunden mit der zweiten Kammer ist, welche leer ist, zum Aufnehmen einer Verdünnungslösung aus einer Verdünnungslösungsquelle; und
einen Verbindungsschlauch, der strömungsverbunden mit der zweiten Kammer ist, wobei die Verdünnungslösung über den Verdünnungslösungsschlauch in die zweite Kammer eingefüllt werden kann, um die abziehbare Schweißnaht zu öffnen, sodass das Peritonealdialysekonzentrat verdünnt oder gelöst wird, um über den Verbindungsschlauch eine für eine Peritonealdialysebehandlung geeignete Peritonealdialyselösung zu erhalten.

## Revendications

1. Concentré de dialyse péritonéale comprenant un concentré tout-en-un, le concentré tout-en-un contenant au moins un sel électrolytique, au moins un agent tampon, au moins un agent osmotique et un agent d'ajustement du pH, dans lequel l'agent osmotique comprend au moins l'un parmi le dextrose, l'icodextrine, les polymères de glucose, le sorbitol ou le fructose, et dans lequel en outre le concentré tout-en-un est configuré pour être dilué afin d'obtenir la solution de dialyse péritonéale ayant un pH dans une plage de 4,0 à 8,0 et convenant à un traitement de dialyse péritonéale,
et dans lequel la solution de dilution est de l'eau pour préparation injectable ou de l'eau pour préparation injectable générée directement à partir d'eau pure ou d'eau osmosée, **caractérisé en ce que**
le pH du concentré tout-en-un est dans une plage de 4,0 à 8,0.

2. Concentré de dialyse péritonéale selon la revendication 1, dans lequel l'agent d'ajustement du pH est l'acide chlorhydrique.

3. Concentré de dialyse péritonéale selon la revendication 1 ou 2, dans lequel :
le sel électrolytique comprend un sel sélectionné dans un groupe constitué d'un sel de sodium, d'un sel de calcium, d'un sel de magnésium et de tous mélanges de ceux-ci ; et/ou
l'agent tampon est un agent tampon alcalin comprenant un agent sélectionné dans un groupe constitué du lactate de sodium, du bicarbonate de sodium et de tous mélanges de ceux-ci ;
et/ou
l'agent osmotique comprend un agent sélectionné dans un groupe constitué des acides aminés, de la glycérine cristalline à petite molécule, d'un gel macromoléculaire, d'un polymère cationique, d'un polypeptide et de tous mélanges de ceux-ci.

4. Concentré de dialyse péritonéale selon l'une quelconque des revendications 1 à 3, dans lequel :
le concentré de dialyse péritonéale peut être dilué de 2 à 20 fois afin d'obtenir la solution de dialyse péritonéale.

5. Concentré de dialyse péritonéale selon la revendication 4, dans lequel le concentré de dialyse péritonéale peut être dilué de 8 à 12 fois.

6. Concentré de dialyse péritonéale selon l'une quelconque des revendications 1 à 5, dans lequel
la solution de dialyse péritonéale peut être utilisée pour une dialyse péritonéale continue ambulatoire ou un traitement de dialyse péritonéale automatisée.

7. Ensemble pour une dialyse péritonéale continue ambulatoire ou une dialyse péritonéale automatisée, dans lequel l'ensemble comprend une poche de dialyse péritonéale à chambre unique, qui contient le concentré de dialyse péritonéale selon l'une quelconque des revendications 1 à 6, la poche de dialyse péritonéale à chambre unique comprenant :
une chambre unique destinée à contenir un concentré de dialyse péritonéale ;
une tubulure de solution de dilution reliée fluidiquement à la chambre pour recevoir une solution de dilution provenant d'une source de solution de dilution ; et
une tubulure de raccordement reliée fluidiquement à la chambre,
dans lequel la solution de dilution peut être introduite dans la chambre via la tubulure de solution de dilution afin de diluer ou de dissoudre le concentré de dialyse péritonéale et d'obtenir une solution de dialyse péritonéale convenant à un traitement de dialyse péritonéale via la tubulure de raccordement.

8. Ensemble pour une dialyse péritonéale continue ambulatoire ou une dialyse péritonéale automatisée, dans lequel l'ensemble comprend une poche de dialyse péritonéale à double chambre comprenant :
une première chambre contenant le concentré de dialyse péritonéale selon l'une quelconque des revendications 1 à 6 ;
une seconde chambre séparée de la première chambre par une ligne de soudure pelable ; une tubulure de solution de dilution reliée fluidiquement à la seconde chambre, qui est vide, pour recevoir une solution de dilution provenant d'une source de solution de dilution ; et
une tubulure de raccordement reliée fluidiquement à la seconde chambre,
dans lequel la solution de dilution peut être introduite dans la seconde chambre via la tubulure de solution de dilution afin de rompre la ligne de soudure pelable de telle sorte que le concentré de dialyse péritonéale soit dilué ou dissous pour obtenir une solution de dialyse péritonéale convenant à un traitement de dialyse péritonéale via la tubulure de raccordement.
